Europäisches Patentamt

European Patent Office

Office européen des brevets

(11) Publication number: **0 594 109 A1**

## EUROPEAN PATENT APPLICATION

(21) Application number: **93116804.1**

(22) Date of filing: **18.10.93**

(51) Int. Cl.⁵: **C07C 69/54**, C08F 220/28, C08F 8/00, C09D 133/06, C08G 18/80

(30) Priority: **19.10.92 US 962561**

(43) Date of publication of application:
**27.04.94 Bulletin 94/17**

(84) Designated Contracting States:
**AT BE CH DE DK ES FR GB GR IE IT LI LU MC NL PT SE**

(71) Applicant: **UNION CARBIDE CHEMICALS & PLASTICS TECHNOLOGY CORPORATION**
**39 Old Ridgebury Road**
**Danbury, Connecticut 06817-0001(US)**

(72) Inventor: **Argyropoulos, John Nicholas**
**35 Michael Street**
**Scott Depot, W. Virginia 25560(US)**
Inventor: **Kilker, Brian Lee**
**2 Windsong Acres**
**Winfield, W. Virginia 25313(US)**
Inventor: **Koleske, Joseph Victor**
**1513 Brentwood Road**
**Charleston, W. Virginia 25314(US)**
Inventor: **Lewis, Jeffrey Michael Owen**
**29746 Briarton**
**Farmington Hills, Michigan 48331(US)**

(74) Representative: **Brandes, Jürgen, Dr. rer. nat. et al**
**Wuesthoff & Wuesthoff**
**Patent- und Rechtsanwälte**
**Schweigerstrasse 2**
**D-81541 München (DE)**

(54) **Hindered-hydroxyl functional (meth)acrylate esters, copolymers containing them and compositions including same.**

(57) This invention relates to hindered-hydroxyl functional (meth)acrylate compounds and derivatives thereof and to processes for the preparation thereof. This invention also relates to hindered-hydroxyl functional (meth)-acrylate-containing copolymers and to compositions including same. The hindered-hydroxyl functional (meth)-acrylate-containing copolymers have utility in coatings, adhesives, inks, sealants, as well as in other end uses.

EP 0 594 109 A1

## Brief Summary of the Invention

### Technical Field

This invention relates to hindered-hydroxyl functional (meth)acrylate compounds and derivatives thereof and to processes for the preparation thereof. The hindered-hydroxyl functional (meth)acrylate compounds are useful as copolymerizable monomers in various polymerization methods. This invention also relates to hindered-hydroxyl functional (meth)acrylate-containing copolymers and to compositions including same. The hindered-hydroxyl functional (meth)acrylate-containing copolymers are useful in preparing coatings, adhesives, inks and sealants that can be cured with a variety of crosslinking agents.

### Background of the Invention

Although (meth)acrylic coatings are known to have very good weathering characteristics, they are subject to deterioration by etching under both high and low pH conditions. Such etching first manifests itself as permanent spotting which is unpleasing to the eye. After a period of time that is often dependent on temperature, there is removal or other deterioration of the coating in the spotted areas and then further deterioration in other areas and finally failure of the coating in a functional sense. Such etching takes place during the drying of droplets of water containing relatively small amounts of acidic or alkaline moieties from the substrate. As the droplet decreases in volume, the pH decreases to very low values in the case of acid moieties and increases to very high values in the case of alkaline moieties. Under these conditions, relatively rapid attack of the substrate occurs.

The coatings industry is actively seeking coatings with improved hydrolytic resistance particularly at high and low pH values over that of the current acrylics. Such improved resistance would result in coatings that have enhanced resistance to hostile environment as such as those caused by acid rain, by air-borne chemicals, and by cleaners used for dirt removal.

Another need in the field of coatings is low viscosity coating formulations that will allow higher application solids to be achieved and maintain or improve final cured coating performance characteristics. The requirement for such high solids coating formulations is driven by both federal and state regulations as well as a desire to decrease any impact volatile organic solvents may have on the environment. Coatings that have high solids can have an unbalanced property profile that is usually related to the low molecular weight oligomers/polymers that must be used to obtain a sufficiently low viscosity in formulated coating systems that have low volatile organic content. Coatings with high solids, low volatile organic content, and improved physical and chemical characteristics result in a decreased environmental impact caused by volatile solvents, decreased energy requirements for volatile solvent manufacture and volatilization, and a longer service life which requires less replacement and thus conserves both energy and natural resources.

### Disclosure of the Invention

This invention relates in part to hindered-hydroxyl functional meth(acrylate) compounds represented by the formula:

$$R_1 R_2 C = C(R_3) - C(O) - O - R_4 \qquad (I)$$

wherein:

$R_1$, $R_2$ and $R_3$ are the same or different and are hydrogen or a substituted or unsubstituted monovalent hydrocarbon residue, preferably an alkyl group of 1 to 3 carbon atoms;

$R_4$ is a substituted or unsubstituted monovalent hydrocarbon residue represented by the formula:

$$-(CR_5 R_6)_v - (CR_7 R_8)_w - (C(OH)R_9)_x - (CR_{10}R_{11})_y - (CR_{12}R_{13})_z - R_{14}$$

wherein:

each $R_5$, $R_6$, $R_7$, $R_8$, $R_9$, $R_{10}$, $R_{11}$, $R_{12}$ and $R_{13}$ are the same or different and are hydrogen or a substituted or unsubstituted monovalent hydrocarbon residue;

$R_{14}$ is hydrogen, hydroxyl or a substituted or unsubstituted monovalent hydrocarbon residue provided $R_{14}$ is hydroxyl when x is value of 0 and $R_{14}$ is other than hydroxyl when x is a value of 1;

each of v, w, y and z is a value of from 0 to about 5 and the sum of v + w + x + y + z is a value of from about 3 to about 15; and

2

x is a value of 0 or 1;

provided (i) at least one of w and y is a value other than 0 when x is a value of 1; (ii) at least one of $R_7$, $R_8$, $R_9$, $R_{10}$ and $R_{11}$ is other than hydrogen when x is a value of 1; and (iii) at least one of $R_{12}$ and $R_{13}$ is other than hydrogen when x is a value of 0.

This invention also relates in part to processes for preparing hindered-hydroxyl functional (meth)acrylate compounds.

This invention further relates in part to copolymers comprising the reaction product of (a) one or more hindered-hydroxyl functional (meth)acrylate monomers and (b) at least one other monomer copolymerizable therewith, said copolymer having (i) a number average molecular weight of less than about 12,000, preferably less than about 10,000, and most preferably less than about 7500, (ii) a glass transition temperature of from -30°C to about 100°C, preferably from about -20°C to about 65°C, and most preferably from about 0°C to about 45°C, and (iii) a styrene concentration of from 0 to less than about 20 weight percent, and said hindered-hydroxyl functional (meth)-acrylate monomer having a diacrylate content of less than about 5 weight percent.

This invention also relates in part to compositions comprising (a) the copolymer described above, (b) a crosslinking agent, and (c) optional ingredients. Such compositions include, for example, coatings, adhesives, inks, sealants and the like.

This invention further relates in part to a method of protecting a surface from corrosion when exposed to a hostile environment, e.g., an acidic or alkaline environment, which comprises applying to the surface a composition described above, and curing said composition.

Detailed Description

The hindered-hydroxyl functional (meth)acrylate compounds of this invention can be prepared, for example, by (i) direct esterification of appropriate diol compound with methacrylic acid or acrylic acid, (ii) reaction of appropriate diol compound with methacrylic anhydride or acrylic anhydride, and (iii) transesterification of appropriate diol compound with alkyl methacrylate or alkyl acrylate, e.g., methyl methacrylate, methyl acrylate, ethyl methacrylate, ethyl acrylate, propyl methacrylate, propyl acrylate, and the like.

The direct esterification of appropriate diol compound with methacrylic acid or acrylic acid can be conducted at a temperature of from about 100°C to 300°C for a period of about 1 hour to about 7 days with the longer time being used at the lower temperature, preferably from about 125°C to about 200°C 1 hour to about 5 days, and more preferably at about 125°C to 150°C for about 1 hour to about 48 hours. Direct esterifications are often carried out at reflux temperature which is dependent on the boiling point temperature of the solvent used for a period of about 6 to about 8 hours. Suitable solvents include benzene, xylene, toluene and the like. One or more stabilizers such as hydroquinone, methoxyhydroquinone, phenothiazine and the like should be used to prevent polymerization.

The direct esterification reaction can be conducted over a wide range of pressures ranging from atmospheric pressure to superatmospheric pressures, e.g., from about 1 atmosphere to about 100 atmospheres or greater. It is preferable to conduct the direct esterification reaction at pressures of from about atmospheric to about 25 atmospheres. The direct esterification reaction is preferably effected in the liquid or vapor states or mixtures thereof.

The molar ratio of appropriate diol compound to methacrylic acid or acrylic acid in the direct esterification reaction is not narrowly critical and can range from about 0.05:1 or less to about 50:1 or greater, preferably from about 0.1:1 to about 10:1.

The reaction of appropriate diol compound with methacrylic anhydride or acrylic anhydride can be conducted at a temperature of from about 0°C to 150°C for a period of about 1 hour to about 7 days with the longer time being used at the lower temperature, preferably from about 15°C to about 100°C for about 1 hour to about 5 days, and more preferably at about 25°C to 50°C for about 1 hour to about 48 hours. One or more stabilizers such as hydroquinone, methoxyhydroquinone, phenothiazine, and the like should be used to prevent polymerization.

The reaction of appropriate diol compound with methacrylic anhydride or acrylic anhydride can be conducted over a wide range of pressures ranging from atmospheric pressure to superatmospheric pressures, e.g., from about 1 atmosphere to about 100 atmospheres or greater. It is preferable to conduct the reaction at pressures of from about atmospheric to about 25 atmospheres. The reaction is preferably effected in the liquid or vapor states or mixtures thereof.

The molar ratio of appropriate diol compound to methacrylic anhydride or acrylic anhydride in the reaction is not narrowly critical and can range from about 0.05:1 or less to about 50:1 or greater, preferably

EP 0 594 109 A1

from about 0.1:1 to about 10:1.

The transesterification of appropriate diol compound with alkyl methacrylate or alkyl acrylate can be conducted at a temperature of from about -20°C to 250°C for a period of about 10 minutes to about 7 days with the longer time being used at the lower temperature, preferably from about 0°C to about 200°C for about 1 hour to about 5 days, and more preferably at about 0°C to 180°C for about 1 hour to about 48 hours. One or more stabilizers such as hydroquinone, methoxyhydroquinone, phenothiazine and the like should be used to prevent polymerization.

The transesterification reaction can be conducted over a wide range of pressures ranging from atmospheric pressure to superatmospheric pressures, e.g., from about 1 atmosphere to about 100 atmospheres or greater. It is preferable to conduct the transesterification reaction at pressures of from about atmospheric to about 25 atmospheres. The transesterification reaction is preferably effected in the liquid or vapor states or mixtures thereof.

The molar ratio of appropriate diol compound to alkyl methacrylate or alkyl acrylate in the transesterification reaction is not narrowly critical and can range from about 0.05:1 or less to about 50:1 or greater, preferably from about 0.1:1 to about 10:1.

Illustrative of suitable diol compounds useful in the hindered-hydroxyl functional (meth)acrylate compound preparation processes of this invention include, for example, 2-ethyl-1,3-hexanediol, 2-methyl-1,3-pentanediol, 2-propyl-1,3-heptane diol, 2-ethyl-1,3-heptane diol, 2-ethyl-1,3-propane diol, 2-i-propyl-3-i-butyl-1,3-propane diol, 2-i-propyl-3-methyl-1,3-propane diol, 1-i-butyl-1,3-propanediol, 1-methyl-2-butyl-1,3-propane diol, 2,2,4-trimethyl-1,3-pentane diol, and the like. Preferred hindered-hydroxyl functional (meth)acrylate compounds of this invention are obtained from 2,2,4-trimethyl-1,3-pentane diol, 2-methyl-1,3-pentane diol and 2-ethyl-1,3-hexane diol. The diol compounds can be prepared by conventional methods such as aldol condensations, the reaction of formaldehyde with ketones, or other suitable methods.

Optionally the transesterification reaction mass may contain a transesterification catalyst. Illustrative of suitable transesterification catalysts are potassium bicarbonate, sodium bicarbonate, potassium thiocyanate, barium thiocyanate, calcium triocyanate, cobalt thiocyanate, sodium thiocyanate, zinc thiocyanate, other salts of weak acids such as sodium acetate, lithium acetate, calcium acetate, zinc acetate, and other metal salts of acetic acid and carbonic acid, alkali metal alkoxides such as sodium methoxide, potassium methoxide, lithium methoxide, zinc methoxide, calcium methoxide, metal oxalates, and the like. When used, the catalysts can be present in amounts of from about 0.10 mole percent or less to 20 mole percent with it preferred that from about 0.20 mole percent to about 10 mole percent be used. The transesterification catalyst may be added to the reaction mass all at one time, in discrete portions that may be of the same or different size, or in a continuous uniform or non uniform manner over the entire reaction time period or over a portion of the reaction time period.

The products produced by the processes of this invention include hindered-hydroxyl functional (meth)-acrylate compounds, in particular, those hindered-hydroxyl functional (meth)acrylate compounds encompassed within Formula (I) above. These products are useful in a number of ways including imparting excellent physical characteristics, such as water resistance, chemical resistance, resistance to hostile environments such as acid rain, and the like, to coatings, inks, adhesives, and sealants prepared from the hindered-hydroxyl functional (meth)acrylate compounds or derivatives thereof, and as reactive surfactants.

Illustrative hindered-hydroxyl functional (meth)acrylate compounds prepared by the processes of this invention include, for example, 2-ethyl-3-hydroxyhexyl methacrylate, 1-propyl-2-ethyl-3 hydroxypropyl methacrylate, 1-ethyl-2-methyl-3-hydroxypropyl methacrylate, 2-methyl-3-hydroxypentyl methacrylate, 2-propyl-3-hydroxyheptyl methacrylate, 1-butyl-2-propyl-3-hydroxypropyl methacrylate, 2-ethyl-3-hydroxyheptyl methacrylate, 1-butyl-2-ethyl-3-hydroxypropyl methacrylate, 2-propyl-3-hydroxypropyl methacrylate, 2-ethyl-3-hydroxypropyl methacrylate, 1-i-butyl-2-i-propyl-3-hydroxypropyl methacrylate, 2-i-propyl-3-hydroxy-5-methylhexyl methacrylate, 1-methyl-2-i-propyl-3-hydroxypropyl methacrylate, 2-i-propyl-3-methyl-3-hydroxypropyl acrylate, 1-i-butyl-3-hydroxypropyl methacrylate, 3-hydroxy-5-methylhexyl methacrylate, 1-methyl-2-butyl-3-hydroxypropyl methacrylate, 2-butyl-3-hydroxybutyl methacrylate, 1-i-propyl-2,2-dimethyl-3-hydroxypropyl methacrylate, 2,2-dimethyl-3-hydroxy-4-methylpentyl methacrylate, and the like.

For purposes of Formula (I) above, the following compounds defined in (a) through (r) are outside the scope of claim 1 of this invention:

(a) when v, w and z are each a value of 1, x and y are each a value of 0, $R_1$, $R_2$, $R_5$, $R_6$, $R_7$ and $R_8$ are each hydrogen, $R_3$ is hydrogen or methyl, and $R_{14}$ is hydroxyl, then at least one of $R_{12}$ and $R_{13}$ is other than hydrogen or methyl;

(b) when v and z are each a value of 1, w, x and y are each a value of 0, $R_1$ and $R_2$ are each hydrogen, $R_3$ is hydrogen or methyl, one of $R_5$ and $R_6$ is methyl and the other is hydrogen, and $R_{14}$ is hydroxyl, then at least one of $R_{12}$ and $R_{13}$ is other than hydrogen or methyl;

4

(c) when y and z are each a value of 1, v, w and x are each a value of 0, $R_1$ and $R_2$ are each hydrogen, $R_3$ is hydrogen or methyl, $R_{10}$ and $R_{11}$ are each methyl, and $R_{14}$ is hydroxyl, then at least one of $R_{12}$ and $R_{13}$ is other than hydrogen or methyl;

(d) when w, y and z are each a value of 1, v and x are each a value of 0, $R_1$, $R_2$, $R_{10}$ and $R_{11}$ are each hydrogen, $R_3$ is hydrogen or methyl, one of $R_7$ and $R_8$ is methyl and the other is hydrogen, and $R_{14}$ is hydroxyl, then at least one of $R_{12}$ and $R_{13}$ is other than hydrogen or methyl;

(e) when v, w and z are each a value of 1, y is a value of 2, x is a value of 0, $R_1$, $R_2$, $R_3$, $R_5$, $R_6$, $R_7$, $R_8$, $R_{10}$ and $R_{11}$ are each hydrogen, and $R_{14}$ is hydroxyl, then at least one of $R_{12}$ and $R_{13}$ is other than hydrogen or methyl;

(f) when v, w, y and z are each a value of 1, x is a value of 0, $R_1$, $R_2$, $R_5$, $R_6$, $R_7$, $R_8$, $R_{10}$ and $R_{11}$ are each hydrogen, $R_3$ is methyl, and $R_{14}$ is hydroxyl, then at least one of $R_{12}$ and $R_{13}$ is other then hydrogen or ethyl;

(g) when v, w and z are each a value of 1, x and y are each a value of 0, $R_1$, $R_2$, $R_3$, $R_7$ and $R_8$ are each hydrogen, $R_5$ and $R_6$ are each methyl, and $R_{14}$ is hydroxyl, then at least one of $R_{12}$ and $R_{13}$ is other than hydrogen or methyl;

(h) when v, w, y and z are each a value of 1, x is a value of 0, $R_1$, $R_2$, $R_3$, $R_7$, $R_8$, $R_{10}$ and $R_{11}$ are each hydrogen, one of $R_5$ and $R_6$ is methyl and the other is hydrogen, and $R_{14}$ is hydroxyl, then at least one of $R_{12}$ and $R_{13}$ is other than hydrogen or methyl;

(i) when v, w and z are each a value of 1, x and y are each a value of 0, $R_1$, $R_2$, $R_3$, $R_7$ and $R_8$ are each hydrogen, $R_5$ and $R_6$ are each methyl, and $R_{14}$ is hydroxyl, then at least one of $R_{12}$ and $R_{13}$ is other than hydrogen or methyl;

(j) when v and z are each a value of 1, w, x and y are each a value of 0, $R_1$, $R_2$, $R_5$ and $R_6$ are each hydrogen, $R_3$ is hydrogen or methyl, and $R_{14}$ is hydroxyl, then at least one of $R_{12}$ and $R_{13}$ is other than hydrogen or phenyl;

(k) when v, w and z are each a value of 1, x and y are each a value of 0, $R_1$, $R_2$, $R_5$ and $R_6$ are each hydrogen, $R_3$, $R_7$ and $R_8$ are each methyl, and $R_{14}$ is hydroxyl, then at least one of $R_{12}$ and $R_{13}$ is other than hydrogen or propyl;

(l) when v, w, x and z are each a value of 1, y is a value of 0, $R_1$, $R_2$, $R_5$, $R_6$ and $R_9$ are each hydrogen, $R_3$ is hydrogen or methyl, and $R_7$ and $R_8$ are each methyl, then at least one of $R_{12}$, $R_{13}$ and $R_{14}$ is other than hydrogen or methyl;

(m) when v, w, y and z are each a value of 1, x is a value of 0, $R_1$, $R_2$, $R_3$, $R_7$, $R_8$, $R_{10}$ and $R_{11}$ are each hydrogen, $R_5$ and $R_6$ are each methyl, and $R_{14}$ is hydroxyl, then at least one of $R_{12}$ and $R_{13}$ is other than hydrogen or methyl;

(n) when v, w and z are each a value of 1, y is a value of 3, x is a value of 0, $R_1$, $R_2$, $R_5$, $R_6$, $R_7$, $R_8$, $R_{10}$ and $R_{11}$ are each hydrogen, $R_3$ is methyl, and $R_{14}$ is hydroxyl, then at least one of $R_{12}$ and $R_{13}$ is other than hydrogen or ethyl;

(o) when v and z are each a value of 1, w, x and y are each a value of 0, $R_1$, $R_2$, $R_3$, $R_5$ and $R_6$ are each hydrogen, and $R_{14}$ is hydroxyl, then at least one of $R_{12}$ and $R_{13}$ is other than hydrogen, ethyl or butyl;

(p) when w, x and z are each a value of 1, v and y are each a value of 0, $R_1$, $R_2$, $R_3$, $R_7$, $R_8$ and $R_9$ are each hydrogen, then at least one of $R_{12}$, $R_{13}$ and $R_{14}$ is other than hydrogen or phenyl;

(q) when v and z are each a value of 1, w, x and y are each a value of 0, $R_1$, $R_2$, $R_3$, $R_5$ and $R_6$ are each hydrogen, and $R_{14}$ is hydroxyl, then at least one of $R_{12}$ and $R_{13}$ is other than hydrogen, methyl or phenyl; and

(r) when v, w and z are each a value of 1, y is a value of 9, x is a value of 0, $R_1$, $R_2$, $R_3$, $R_5$, $R_6$, $R_7$, $R_8$, $R_{10}$ and $R_{11}$ are each hydrogen, and $R_{14}$ is hydroxyl, then at least one of $R_{12}$ and $R_{13}$ is other than hydrogen or methyl.

The hindered-hydroxyl functional (meth)acrylate compounds produced by the processes of this invention can be separated by distillation. For example, a crude reaction product can be subjected to a distillation-separation at atmospheric or reduced pressure through a packed distillation column. Reactive distillation may be useful in conducting certain reactions of this invention.

The hindered-hydroxyl functional (meth)acrylate compound preparation processes of this invention may be carried out using, for example, a fixed bed reactor, a fluid bed reactor, or a slurry reactor. The optimum size and shape of the catalysts will depend on the type of reactor used. In general, for fluid bed reactors, a small, spherical catalyst particle is preferred for easy fluidization. With fixed bed reactors, larger catalyst particles are preferred so the back pressure within the reactor is kept reasonably low.

The hindered-hydroxyl functional (meth)acrylate compound preparation processes of this invention can be conducted in a batch or continuous fashion, with recycle of unconsumed starting materials if required. The reaction can be conducted in a single reaction zone or in a plurality of reaction zones, in series or in parallel or it may be conducted batchwise or continuously in an elongated tubular zone or series of such zones. The materials of construction employed should be inert to the starting materials during the reaction and the fabrication of the equipment should be able to withstand the reaction temperatures and pressures. Means to introduce and/or adjust the quantity of starting materials or ingredients introduced batchwise or continuously into the reaction zone during the course of the reaction can be conveniently utilized in the processes especially to maintain the desired molar ratio of the starting materials. The reaction steps may be effected by the incremental addition of one of the starting materials to the other. Also, the reaction steps can be combined by the joint addition of the starting materials. When complete conversion is not desired or not obtainable, the starting materials can be separated from the product, for example by distillation, and the starting materials then recycled back into the reaction zone.

The hindered-hydroxyl functional (meth)acrylate compound preparation processes are conducted for a period of time sufficient to produce the hindered-hydroxyl functional (meth)acrylate compounds. The exact reaction time employed is dependent, in part, upon factors such as temperature, nature and proportion of starting materials, and the like. The reaction time will normally be within the range of from about one-half to about 100 hours or more, and preferably from less than about one to about ten hours.

The hindered-hydroxyl functional (meth)acrylate compound preparation process may be conducted in either glass lined, stainless steel or similar type reaction equipment. The reaction zone may be fitted with one or more internal and/or external heat exchanger(s) in order to control undue temperature fluctuations, or to prevent any possible "runaway" reaction temperatures.

The hindered-hydroxyl functional (meth)acrylate compounds produced by the processes of this invention can undergo further reaction(s) to afford desired derivatives thereof. Such permissible derivatization reactions can be carried out in accordance with conventional procedures known in the art. Illustrative derivatization reactions include, for example, esterification, etherification, alkoxylation, amination, alkylation, hydrogenation, dehydrogenation, reduction, acylation, condensation, carboxylation, carbamoylation, oxidation, silylation and the like, including permissible combinations thereof. This invention is not intended to be limited in any manner by the permissible derivatization reactions or permissible derivatives of hindered-hydroxyl functional (meth)acrylate compounds.

More particularly, the hindered-hydroxyl functional (meth)acrylate compounds of this invention can undergo any of the known reactions of hydroxyl groups illustrative of which are reactions with acyl halides to form esters; with ammonia, a nitrile, or hydrogen cyanide to form amines; with alkyl acid sulfates to form disulfates; with carboxylic acids and acid anhydrides to form esters and polyesters; with alkali metals to form salts; with ketenes to form esters; with acid anhydrides to form carboxylic acids; with oxygen to form aldehydes and carboxylic acids; ring-opening reactions with lactones, tetrahydrofuran, and alkylene oxides such as ethylene oxide, propylene oxide, epichlorohydrin; dehydrogenation to form aldehydes, isocyanates to form urethanes, and the like.

The hindered hydroxyl-functional (meth)acrylate compounds of the invention are useful in the formation of intermediates such as copolymers/oligomers/polymers for formulation with crosslinking agents; for preparation of alkylene oxide and lactone adducts that are useful as surfactants and/or intermediates; for preparation of urethane (meth)acrylates; for preparation of carboxyl-terminated, carbamoyl-terminated, and isocyanate-terminated (meth)acrylate adducts; and the like.

The copolymers of this invention can be prepared by conventional free-radical, chain-transfer polymerization techniques which are well known to those skilled in the art. As used herein, the term "copolymer is contemplated to include oligomers and polymers. Chain transfer agents are compounds such as the alkyl mercaptans illustrative of which is tertiary-dodecyl mercaptan and the like; hydroxyl containing compounds such as alcohols including propanol, isopropanol, butanols, pentanol, hexanol; diols including ethylene glycol, 1,3-propane diol, 1,4-butane diol, 2,3-butane diol, pentane diols, hexane diols, and diols described herein; hydroxyl alkyl acrylates as described herein; and the like. When used, chain transfer agents are present in an amount up to about 5 percent by weight or greater based on weight of the free-radical polymerizable monomers.

Illustrative hindered-hydroxyl functional (meth)acrylate monomers useful in the copolymers of this invention include, for example, 2-ethyl-3-hydroxyhexyl methacrylate, 1-propyl-2-ethyl-3 hydroxypropyl methacrylate, 1-ethyl-2-methyl-3-hydroxypropyl methacrylate, 2-methyl-3-hydroxypentyl methacrylate, 2,2,4-trimethyl-3-hydroxypentyl methacrylate, 2-propyl-3-hydroxyheptyl methacrylate, 1-butyl-2-propyl-3-hydroxypropyl methacrylate, 2-ethyl-3-hydroxyheptyl methacrylate, 1-butyl-2-ethyl-3-hydroxypropyl methacrylate, 2-propyl-3-hydroxypropyl methacrylate, 2-ethyl-3-hydroxypropyl methacrylate, 1-i-butyl-2-i-propyl-3-hydroxypropyl methacrylate, 2-i-propyl-3-hydroxy-5-methylhexyl methacrylate, 1-methyl-2-i-propyl-3-hydroxypropyl methacrylate, 2-i-propyl-3-methyl-3-hydroxypropyl acrylate, 1-i-butyl-3-hydroxypropyl methacrylate, 3-hydroxy-5-methylhexyl methacrylate, 1-methyl-2-butyl-3-hydroxypropyl methacrylate, 2-butyl-3-hydroxybutyl methacrylate, 1-i-propyl-2,2-dimethyl-3-hydroxypropyl methacrylate, 2,2-dimethyl-3-hydroxy-4-methylpentyl methacrylate, and the like. Preferred hindered-hydroxyl functional (meth)acrylate monomers useful in this invention are obtained from 2,2,4-trimethyl-1,3-pentane diol, 2-methyl-1,3-pentane diol and 2-ethyl-1,3 hexane diol. Suitable hindered-hydroxyl functional (meth)acrylate monomers useful in preparing the copolymers of this invention include, but are not limited to, those represented by the Formula (I) above.

The hindered-hydroxyl functional (meth)acrylate monomers useful in this invention impart excellent physical characteristics, such as water resistance, chemical resistance, resistance to hostile environments such as acid rain, and the like, to coatings, inks, adhesives, and sealants prepared from the hindered-hydroxyl functional (meth)acrylate monomers or derivatives thereof.

Suitable other monomers copolymerizable with the hindered-hydroxyl functional (meth)acrylate monomers include one or more monoethylenically and/or multiethylenically unsaturated copolymerizable monomers, for example, one or more other (meth)acrylates, hydroxyalkyl (meth)acrylates, N-(alkoxymethyl)-acrylamides such as N-(iso-butoxymethyl)-acryl-amide and N-methylol-acrylamide, vinyl compounds and the like. The other copolymerizable monomer can be the same as or different from the hindered-hydroxyl functional (meth)acrylate monomer.

Illustrative of the other (meth)acrylates include, for example, acrylic acid, methacrylic acid, the esters of acrylic and methacrylic acid such as the various methyl, ethyl, propyl, butyl, pentyl, hexyl, octyl, decyl, dodecyl, and the like acrylates including the various isomers of these and other listed compounds; bornyl, isobornyl, norbornyl and isonorbornyl acrylate; unsaturated carbamoyloxy carboxylates such as those described in U.S. Patent No. 3,674,838; 3-methacryloxypropyltris(trimethyl-siloxy)silane and 3-acryloxypropyltris(trimethyl-siloxy)silane; dicyclopentenylacrylate; hydroxyethyl acrylate, hydroxypropyl acrylate, hydroxyhexyl acrylates, hydroxydecyl acrylates, caprolactone acrylates which are the product of reacting an e-caprolactone with a hydroxyalkyl-acrylate and which have both acrylate and hydroxyl functionality, including carbamoyloxyalkanoyloxyalkyl (meth)acrylates, ethoxylated and propoxylated acrylates which are the product of reacting an alkylene oxide illustrative of which are ethylene oxide, propylene oxide, and the like, with an hydroxyalkylacrylate; cyclohexyl acrylate, 2-phenoxyethyl acrylate, glycidyl acrylate, and the like.

Illustrative vinyl compounds include, for example, styrene, vinyl cyclohexane, vinyl cyclohexene, vinyl cyclooctane, N-vinylpyrrolidone, vinylpyridines, vinyl imidazole, vinyl naphthalene, acrylonitrile, methacrylonitrile, vinyl chloride, vinyl fluoride, vinyl bromide, vinylidine fluoride, vinylidine chloride, 5-vinyl-2-norbornene and other vinyl norbornenes; vinyl esters such as vinyl acetate, vinyl trifluoroacetate, vinyl propionates, vinyl butyrates, vinyl penanoates, vinyl 2-ethylhexanoate, vinyl nonanoates, vinyl decanoates, vinyl neonanoate, vinyl necodecanoate, vinyl neopentanoate and the like; vinyl ethers such as vinyl alcohol which is formed by the hydrolysis of vinyl acetate, vinyl acetate, vinyl propionates, vinyl triethylene glycol and the like; vinylacetic acid, 3-vinylbenzyl chloride, 4-vinylbiphenyl, vinyl carbazole, vinyl chloroformate, vinyl crotanate, vinyltrimethylsilane, vinyltrimethoxysilane, vinylferrocene, vinyltributyltin, vinyl sulfonic acid, and the like. Included within the definition of vinyl compounds is maleic anhydride, maleic acid, and maleate esters and half esters.

As indicated above, the copolymers/oligomers of this invention have (i) a number average molecular weight of less than about 12,000, preferably less than about 10,000, and most preferably less than about 7500, (ii) a glass transition temperature of from -30°C to about 100°C, preferably from about -20°C to about 65°C, and most preferably from about 0°C to about 45°C, and (iii) a styrene concentration of from 0 to less than about 20 weight percent, and the hindered-hydroxyl functional (meth)acrylate monomer has a diacrylate content of less than about 5 weight percent. These properties are important in that the compositions of this invention containing the hindered-hydroxyl functional (meth)acrylate-containing copolymers can exhibit excellent physical characteristics such as water resistance, chemical resistance, resistance to hostile environments such as acid rain and air-borne chemicals, corrosion resistance, acid etch resistance, alkaline etch resistance, low surface tension, low viscosity that will allow higher application solids to be achieved and maintain or improve final cured coating performance characteristics, and the like.

The copolymers/oligomers of this invention can be prepared by a variety of polymerization techniques illustrative of which are solution polymerization, aqueous emulsion, dispersion, or suspension polymerization, bulk polymerization, nonaqueous emulsion, dispersion, or suspension polymerization, and the like. Said polymerizations can be effected in a variety of reactors illustrative of which are stirred batch reactor, tubular reactors, and the like and can be made of various materials of construction all of which are known to those skilled in the art of conducting such polymerizations.

In a particular embodiment of this invention, the hindered-hydroxyl functional copolymers of the invention are prepared by the following process. This process is particularly useful because when the hindered-hydroxyl functional (meth)acrylate monomer described is prepared, it is difficult to separate the

7

starting diol from the various acrylates formed. This is because both monomeric isomers and the diol have very similar boiling points. To circumvent this difficulty, the reaction is usually carried to a high degree of completion that will minimize the amount of unreacted diol in the final reaction mass; however, this also increases the amount of undesirable diacrylate in the final product. The undesirable diacrylate must be removed by distillation so it does not cause premature crosslinking during preparation of the copolymer/oligomer of the invention. However, if the reaction of (meth)acrylic anhydride, (meth)acrylic acid, or lower alkyl(meth)acrylate is carried to only a low degree of conversion of about 50 percent or less and the excess diol is not removed before carrying out the polymerization, the amount of monoacrylate relative to diacrylate can be maximized and the final product improved. To conduct the process, appropriate diol compounds needed to form the hindered-hydroxyl functional (meth)acrylate monomers and (meth)acrylic anhydride, (meth)acrylic acid, or lower alkyl(meth)acrylate are combined in an approximately 2/1 mole ratio and allowed to react to the point at which there has been about a 50 percent by weight conversion to the appropriate mono(meth)acrylate with only small, less than about 2 percent, quantities of diacrylate formed. This point in the reaction is determined by analysis. This mixture is then combined with one or more monomers copolymerizable with the hindered-hydroxyl functional (meth)acrylate, and the polymerization to the copolymer/oligomer of the invention is carried out. After the copolymer has been prepared, it is separated from the excess diol by vacuum distillation of the diol.

In a particular embodiment of this invention, the hindered-hydroxyl functional copolymers of the invention are modified by reacting all or a portion of the hydroxyl groups pendant on the polymer with a monoisocyanate including cyanic acid and cyanogen chloride to form carbamoyloxyalkanoyloxyalkyl containing copolymers. Included in the hydroxyl groups that can be modified are the hindered hydroxyl groups and those introduced by means of other copolymerizable ethylenically unsaturated monomers such as hydroxyethyl (meth)acrylate, hydroxypropyl (meth)acrylates, the caprolactone acrylates all of which are mentioned above and vinyl alcohol that can be obtained by hydrolysis of vinyl acetate or other vinyl esters, and the like. To form these copolymers, the copolymers of the invention are reacted with monoisocyanates in which the reaction between the hydroxyl group and isocyanate may be represented as follows:

$$-OH \ + \ R_{15}\text{-NCO} \rightarrow -O\text{-CO-NH-}R_{15}$$

wherein $R_{15}$ is hydrogen or a substituted or unsubstituted monovalent hydrocarbon residue. It is preferred that the modification be carried out in the absence of solvents or other compounds that contain hydroxyl groups which groups would interfere with the desired isocyanate/hydroxyl reaction. If desired, said modification can be carried out on the hindered hydroxyl functional (meth)acrylate prior to copolymerization with the other ethylenically unsaturated monomers.

Suitable isocyantes are hydrogen and substituted or unsubstituted monovalent hydrocarbon monoisocyanates illustrative of which are cyanic acid, methyl isocyanate, ethyl isocyanate, propyl isocyanates, butyl isocyanates, pentyl isocyanates, hexyl isocyanates, heptyl isocyanates, octyl isocyanates, nonyl isocyanates, decyl isocyanates, and higher hydrocarbon isocyanates, chloroethyl isocyanate, chlorobutyoxypropyl isocyanate, phenyl isocyanate, o-, m-, and p- chlorophenyl isocyanates, benzyl isocyanate, naphthyl isocyanate, p-ethylphenyl isocyanate, di-chlorophenyl isocyanates, and the like. The monoisocyanates can be used alone or in admixture to modify the copolymers/oligomers of the invention.

The compositions of this invention are comprised of (a) from about 10 weight percent or less to about 90 weight percent or greater, preferably from about 40 weight percent to about 90 weight percent, of the copolymer of this invention, (b) from about 10 weight percent or less to about 90 weight percent or greater, preferably from about 10 weight percent to about 60 weight percent of a suitable crosslinking agent, and (c) optional ingredients. The compositions of this invention can be produced by conventionally formulating the hindered-hydroxyl functional (meth)acrylate-containing copolymers of this invention with one or more of a variety of crosslinking agents and optional ingredients as described below.

Illustrative crosslinking agents suitable for crosslinking the compositions of this invention include, for example, the aminoplasts, the multifunctional isocyanates, phenolics, cycloaliphatic epoxides, glycidyl epoxides; carbodiimides and polycarbodiimides, which can be used when the copolymer contains carboxylic acid or other acidic functionality; and the like. When they will not interfere with each other, mixtures of the various classes or particular crosslinking agents can be used.

To obtain maximum etch resistance, when the copolymer/oligomer containing hindered-hydroxyl functional acrylates is used said copolymer/oligomer should have an oxygen content of less than about 25 weight percent and when aminoplast crosslinking agents are used, it is preferred that said aminoplasts contain about 90% or more alkylation and when isocyanate crosslinking agents are used, it is preferred that triisocyanates are used and most preferred that triisocyanates containing some cyclic ring structure are used.

Illustrative aminoplast crosslinking agents include, for example, alkoxymelamines, melamine-formaldehydes, ureaformaldehydes, alkylated benzoguanimines, guanyl ureas, guanidines, biguanidines, polyguanidines, and the like including mixtures of these compounds. Illustrative of specific compounds are hexamethoxymethylmelamine, methylated melamine, butylated melamine, methylated/butylated melamine, butylated urea, benzoguanidine, and the like.

Illustrative multifunctional isocyanate crosslinking agents include, for example, 4,4'-diphenylmethane diisocyanate, 4,4'-dicyclohexyl diisocyanate, 2,4- and 2,6-toluene diisocyanate, isophorone diisocyanate, xylidiene diisocyanate, meta- and paratetramethylxylene diisocyanate, hexamethylene diisocyanate, 2,2,4- and 2,4,4- trimethylenehexamethylene diisocyanate, 4,4',4''-triisocyanato triphenylmethane, hexamethylene diisocyanate, biurets of hexamethylene diisocyanate with an average functionality greater than 2, and the like. The particular isocyanates can be used in a nascent or a blocked form with the latter type being preferred when a one-package system with maximized pot life/shelf life is desired.

The phenolic crosslinking agents useful in the practice of this invention include, for example, the soluble, heat-reactive phenols or resoles such as those described in T.S. Carswell, Phenoplasts, pages 9-29, Interscience Publishers Inc., New York (1947) and in J. A. Brydson, Plastics Materials, pages 385-386, D. Van Nostrand Co. Inc., New Jersey (1966). Illustrative of the soluble, heat-reactive phenolic crosslinking agents are monomers and polymers of alkylated phenol-formaldehyde, alkylated cresol-formaldehyde, including methylated phenol-formaldehyde, butylated phenol-formaldehyde, cresol-formaldehyde, and the like as well as the various heat reactive phenolics made by reacting phenol, propyl phenols, butyl phenols, amyl phenols, and/or higher hydrocarbon phenols, o-, m-, and p- cresol, xylenols, and the like, with formaldehyde in the presence of a suitable catalyst such as ammonia, ethylamine, triethylamine, as well as other phenols which are known in the art of making heat reactive phenolics.

Illustrative cycloaliphatic epoxide crosslinking agents include, for example, 3,4-epoxy-cyclohexylmethyl 3,4-epoxycyclohexanecarboxylate, 3,4-epoxy-6-methyl-cyclohexylmethyl 3,4-epoxy-6-methylcyclohexanecarboxylate, vinyl cyclohexane diepoxide, cyclohexane diepoxide, cyclopentadiene diepoxide, limonene diepoxide, V-pinene diepoxide, 3,4-epoxycyclohexyl-5,5-spiro-3,4-epoxycyclohexane m-dioxane, bis(3,4-epoxycyclohexylmethyl)adipate, and the like. Although polyfunctional cycloaliphatic epoxides are preferred as crosslinking agents, small amounts of up to about 25% of monoepoxides can be used in the formulation for such purposes as viscosity reduction. Illustrative of the monoepoxides are limonene monoepoxide, V-pinene monoepoxide, vinyl 3,4-epoxycyclohexane, norbornene monoepoxide, cyclohexane monoepoxide, 3,4-epoxy derivatives of alkoxylated and/or lactone derivatives of tetrahydrobenzyl alcohol, and the like.

Illustrative of the glycidyl epoxide crosslinking agents are the diglycidyl ether of bisphenol A, higher homologs of the diglycidyl ether of bisphenol A, diglycidyl ethers of brominated bisphenol A, 1,4-butanediol diepoxide, epoxy esters, epoxy silanes, epoxy siloxanes, epoxy novolacs, and the like.

In an embodiment of this invention, a copolymer containing an N-(alkoxymethyl)acrylamide such as N-(isobutoxymethyl)acrylamide is used as a crosslinking agent for the hindered-hydroxyl functional (meth)-acrylate-containing copolymers of this invention. Thus, a copolymer can be prepared from (a) an N-(alkoxymethyl)acrylamide and (b) one or more monomers copolymerizable therewith. This copolymer can be combined with any of the copolymers of this invention and optionally an acidic catalyst to prepare coating compositions as described herein.

The formulated, uncured compositions containing the copolymers of the invention and cycloaliphatic epoxides can be cured with ultraviolet light when suitable photoinitiators are included in the formulation. The photoinitiators that can be used are of the onium salt type. The ratio of copolymer to cycloaliphatic epoxide can vary broadly in the photocurable compositions, since a wide variety of hard or soft coatings can be made. However, it is preferred that from about 1 to about 50 parts of the copolymer and from about 50 to 99 parts of the cycloaliphatic epoxide be used in the compositions, and more preferred from about 1 to about 30 parts of the copolymer and from about 70 to about 99 parts of the cycloaliphatic epoxide be used. If desired, polyols can be added to the formulation as well as surfactants and acrylates, particularly multifunctional acrylates. Illustrative of the polyols that can be used include poly-e-caprolactone polyols, polyester polyols, polyoxypropylene polyols, poly(oxypropylene/oxyethylene) polyols, polyoxyethylene polyols, polycarbonate polyols, poly(tetramethylene oxide) polyols, ethylene glycol, 1,4-butanediol, 2-ethyl-3-propyl-1,5-pentanadiol, 1,6-hexanediol, and the like. Illustrative of the acrylates that can be used include 2-ethyl hexyl acrylate, trimethylol-propane triacrylate, 1,6-hexanediol diacrylate, ethoxylated trimethylol-propane triacrylate, propoxylated trimethylolpropane triacrylate, higher functional acrylates, and the like.

Illustrative of the onium salt photoinitiators useful in the protocurable coating compositions of this invention containing mixtures of the copolymers of the invention, cycloaliphatic epoxides, and optional ingredients one can mention one or more of a metal fluoroborate and a complex of boron trifluoride as described in U.S. Patent No. 3,379,653; a bis(perfluoroalkylsulfonyl)methane metal salt, as described in U.S. Patent No. 3,586,616; an aryl diazonium compound as described in U.S. Patent No. 3,708,296; an aromatic onium salt of Group VIa elements as described in U.S. Patent No. 4,058,400; an aromatic onium salt of Group Va elements as described in U.S. Patent No. 4,069,055; a dicarbonyl chelate of a Group IIIa-Va element as described in U.S. Patent No. 4,068,091; a thiopyrylium salt as described in U.S. Patent No. 4,139,655; a Group VIb element in an $MF_6$ anion where M is selected from phosphorous, antimony, and arsenic as described in U.S. Patent No. 4,161,478; an arylsulfonium complex salt as described in U.S. Patent No. 4,231,951; an aromatic iodonium complex salt and an aromatic sulfonium complex salt, as described in U.S. Patent 4,256,828; and a bis(4-diphenylsulfonio)phenyl) sulfide-bis-hexafluorometallic salts such as the phosphate, arsenate, antimonate and the like as described by W. R. Watt and coworkers in J. Polymer Sci.: Polymer Chem. Ed., 22, 1789 (1984). Preferred cationic photoinitiators include the arylsulfonium or aryliodonium complex salts, aromatic sulfonium or iodonium salts of halogen containing complex ions, and aromatic onium salts of Group II, V, and VI elements. Some of such salts are commercially available in a solution form as FX-512, thought to be any arylsulfonium hexafluorophosphate, from 3M Company, CYRACURE UVR-6990 and UVR-6974, arylsulfonium hexafluorophosphate and arylsulfonium hexafluoroantimonate, respectively, from Union Carbide Chemicals and Plastics Company Inc.; UVE-1014 and UVE-1016, arylsulfonium hexafluorophosphate and arylsulfonium hexafluoroantimonate, respectively, from General Electric Company, KI-85, thought to be bis(4-(diphenylsulfonio)phenyl)sulfide-bis-hexafluorophosphate, from Degussa AG; and SP-150 and SP-170, thought to be bis(4-(diphenylsulfonio)phenyl)-sulfide-bis-hexafluoro-phosphate and bis(4-(diphenylsulfonio)phenyl)sulfide-bis-hexafluoroantimonate, respectively, from Asahi Denka Kogyo K.K. The onium salt photoinitiators are used at a concentration of less than about 0.1 weight percent to about 10 weight percent, preferably at concentrations of about 0.3 weight percent to about 5 weight percent of the total composition.

It is preferable that a catalyst be used for curing or crosslinking of certain of the compositions of this invention. Illustrative catalysts for thermal curing of the coating compositions when aminoplasts and cycloaliphatic expoxides are used include, among others, p-toluene sulfonic acid and its salts such as ammonium p-toluene sulfonate, diethylammonium sulfonate, diisopropylammonium p-toluene sulfonate, and the like; dodecylbenzene sulfonic acid and its salts such as ammonium dodecylbenzene sulfonate, diethylammonium dodecylbenzene sulfonate, and the like; phosphoric acid and its salts; dinonylnaphthalene sulfonic acids and their salts such as ammonium dinonylnaphthalene sulfonic acids, dipropylammonium dinonylnaphthalene sulfonic acids; diethylammonium dinonylnaphthalene sulfonic acids, and the like; boron trifluoride etherate; trimelletic acid; triflic acid and its salts such as diethylammonium triflate, ammonium triflate, diisopropylammonium triflate, and the like; and when isocyanates are used include, among others, zinc octanoate, stannous octanoate, dibutyltin dilaurate, amines, and the like. The triflic acid salts are particularly useful when cycloaliphatic epoxides are used as the crosslinking agents since they afford low temperature curing conditions to be used along with very good shelf stability at high solids. These catalysts are used in amounts of from about 0.02 weight percent to about 4 weight percent, preferably from about 0.05 weight percent to about 1.0 weight percent, and most preferably from about 0.1 weight percent to about 0.8 weight percent.

The compositions of this invention can be formulated to contain a variety of additives including antioxidants, ultraviolet light stabilizers; surfactants or other flow and leveling agents illustrative of which are silicone oils, acrylic polymers such as the Modaflow® Polymers available from Monsanto Company, silicone/alkylene oxides, fluorocarbon surfactants, and the like; fillers, pigments, colorants, thickeners; reactive diluents; one or more inert solvents illustrative of which are toluene, pentyl propionate, 1,1,1-trichloroethane, ethoxyethyl acetate, propoxyethyl acetate, ethoxybutyl acetate, butyl acetate, methyl isobutyl ketone, mineral spirits, methyl ethyl ketone, methyl amyl ketone, xylene, and the like; inert polymers, waxes, adhesion promoters; slip agents illustrative of which are the silicone oils, powdered polytetrafluoroethylene and/or polyethylene and the like. The additives can be employed in conventional amounts known in the art.

The coating compositions of this invention can be applied and cured on a variety of substrates known to those skilled in the art of coatings technology. Illustrative of such substrates are steel, treated steel, tin-plated steel, galvanized steel, treated and untreated aluminum, glass, wood, paper, coated or printed paper, epoxy/fiberglass composites, polymers such as poly(ethylene terephthalate), poly(butylene terephthalate), treated polyethylene and polypropylene, vinyl film, vacuum or vapor deposited aluminum, gold, copper, silver, zinc, nickel, tin, and other metals, electroless nickel, copper-nickel alloys and the like, electrodeposited metals such as silver, copper, nickel, chromium, silver-copper alloys, and the like, glass-reinforced unsaturated-polyester/styrene products, and the like. Illustrative application methods include, for example, spraying, brushing, dipping, roll coating or other methods.

As indicated above, the compositions of this invention are useful as coatings, adhesives, inks, sealants and the like. The coating compositions of this invention include, for example, water-borne coatings, solvent-borne coatings, powdered coatings, colored coatings, clear coatings, and the like. The coating compositions of this invention are useful in a variety of applications including industrial, architectural, automotive, outdoor signs, outdoor furniture, appliance coatings, recreational vehicles, boats and the like. A particular attribute of the coating compositions of this invention is their durability in outdoor weathering, i.e., providing protection to substrates from various forms of corrosion and deterioration in a functional sense.

For purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds which can be substituted or unsubstituted.

As used herein, the term "substituted" is contemplated to include all permissible substituents of organic compounds unless otherwise indicated. In a broad aspect, the permissible substituents include acyclic and cyclic, branched and unbranched, carbocyclic and heterocyclic, aromatic and nonaromatic substituents of organic compounds. Illustrative substituents include, for example, alkyl, alkyloxy, aryl, aryloxy, hydroxy, hydroxyalkyl, amino, aminoalkyl, halogen and the like in which the number of carbons can range from 1 to about 20 or more, preferably from 1 to about 12. The permissible substituents can be one or more and the same or different for appropriate organic compounds. This invention is not intended to be limited in any manner by the permissible substituents of organic compounds.

As used herein, the molecular weights were determined by gel permeation chromatography using polystyrene calibration standards.

Certain of the following examples are provided to further illustrate this invention.

Glossary of Terms

Aminoplast 1 - A hexamethoxymelamine commercially available from American Cyanamid as Cymel® 303.

Aminoplast 2 - A methylated/butylated melamine commercially available from Monsanto Company as Resimene® 755.

Blocked Isocyanate 1 - A blocked isocyanate, that is thought to be a methyl ethyl ketone oxime blocked trimer of 4,4'-dicyclohexanemethyl diisocyante, commercially available from Miles, Inc. under the designation Desmodur BL-3174A.

Catalyst 1 - A 40% by weight solution of para-toluene sulfonic acid in methanol.

Catalyst 2 - Dibutyltin dilaurate.

Surfactant 1 - A 25% by weight solution in methyl amyl ketone of a silicone-based surfactant commercially available from Union Carbide Chemicals and Plastics Company Inc. as Silwet® L-7001.

Surfactant 2 - A 25% by weight solution in methyl amyl ketone of a silicone-based surfactant commercially available from Union Carbide Chemicals and Plastics Company Inc. as Silwet® L-77.

Photoinitiator 1 - An aryl sulfonium hexafluoroantimonate photoinitiator that is commercially available from Union Carbide Chemicals and Plastics Company Inc. as Cyracure® UVI-6974.

Double Rubs - Solvent resistance was measured as the number of solvent (methyl ethyl ketone double rubs or acetone double rubs) that were required to cut through the coating. If 100 rubs or more did not cut through the coating, the coating was recorded as >100. To perform the test, the solvent-soaked cloth was rubbed back and forth with hand pressure. A rub back and forth was designated as one "double rub."

Crosshatch Adhesion - Procedure conducted in accordance with ASTM D 3359-87.

Pencil Hardness - Procedure conducted in accordance with ASTM D 3363-74.

60° Gloss - Procedure conducted in accordance with ASTM D 523.

20° Gloss - Procedure conducted in accordance with ASTM D 523.

Impact Resistance. Procedure conducted in accordance with ASTM D 2794-84.

Acid etch resistance - A Fini automatic transfer pipette was used to place a series of 50 micro-liter droplets of sulfuric acid solution at approximately 1/4-inch intervals in two rows along the length of one or more coated panels. Usually two panels were required to provide the length of surface needed to examine the temperature range of 40° to 100°C that were achieved in the gradient temperature oven. Two rows of spots were used for duplication of the test. The coated panels were placed in an end-to-end position on the heating bank of a BYK Chemie gradient temperature oven and the first spots were aligned with the #1 rod which was a 40°C which resulted in the various spots being at temperatures that ranged to 100°C. The sulfuric acid solution droplets, which were of indicated acidity, were allowed to contact the coating for various times at the indicated temperatures. After the desired heating time, the panels were removed from the gradient oven, cooled to room temperature, rinsed thoroughly with distilled water, lightly patted dry, and evaluated.

Evaluation was accomplished by examining the areas that had been covered with the droplets with a 10-power, lighted magnifier. The following points of comparison were observed and recorded for each coating.

a) The lowest temperature spot area with a visible defect in the coating. A "visible defect" is the first sign of any blush, bubbling, yellowing, or other visible change.

b) The lowest temperature spot with a severe defect. A "severe defect" is blistering or complete removal of the coating with the substrate visible. This latter factor means the acidic solution has cut though the coating to the substrate.

c) A scaled 1 to 5 rating of any defect or change occurring specifically in the 50°C, 60°C, and 70°C areas of the coating using the following rating system.

1 - Fail. Coating is cut to the substrate or has severe bubbling.

2 - Severe. Small blister or bubble present in the coating.

3 - Moderate. Pinhole defect or slight change in surface of coating by fingertip feeling or visible loss of gloss.

4 - Slight. Blushing or yellowing of coating with no change by fingertip feeling.

5 - Unchanged. No visible evidence of any effect.

## Examples

Preparation A. A methacrylate ester of 2,2,4-trimethyl-1,3-pentanediol was prepared by placing 1800 grams (12.33 moles) of 2,2,4-trimethyl-1,3-pentanediol (TMPD) in a four-neck, glass reaction flask equipped with a Therm-O-Watch temperature control device, a nitrogen inlet and outlet, a stirrer, and a feeding port. The TMPD was melted and dried by heating to 85°C while flowing dry nitrogen through the reactor for 2 hours. Then 4.0 grams of methoxyhydroquinone, 4.0 gram of phenothiazine, and 2,277 grams (14.79 moles) of freshly distilled methacrylic anhydride were added. While stirring and employing a nitrogen purge, 81.0 grams of distilled pyridiene were added and the reaction mass was heated to and held at 35°C for 1-2 days after which time gas chromatographic analysis indicated that the reaction was complete. Excess methacrylic anhydride was quenched by first adding methanol, and these reaction products as well as methacrylic acid formed during the desired alkylmethacrylate ester formation were removed by washing with water. Gas chromatography, mass spectrometry, and Fourier-transform infrared analyses indicated that both monomethacrylate isomers and the expected diacrylate had formed. The monomethacrylates were separated from the diacrylate by fractional distillation.

Example 1 and Control Examples A and B. Example 1 copolymer/oligomer was prepared from a 300-gram mixture of the Preparation A alkyl monomethacrylate mixture, butyl acrylate, and methyl methacrylate. For comparison purposes, mixtures containing hydroxyethyl methacrylate (Control A) or hydroxypropyl methacrylate (Control B) were used in the same molar amount as the Preparation A methacrylate of Example 1 and approximately equal amounts of butyl acrylate and methyl methacrylate so the total amount of monomer mixture equaled about 300 grams. A chain transfer agent, 3-mercapto-1-propanol, was included in the monomer mixture. The initial pentyl propionate solvent was placed in a 2-liter, four-neck, glass reaction flask equipped with a mechanical stirrer, a Thermo-watch heat controller, a nitrogen sparger, a water-cooled condenser, and 500-milliliter and 125-milliliter addition funnels. A nitrogen sparge was maintained throughout the procedure. The solvent was heated to 125°C, and the monomer mixture was fed by means of a piston pump to the flask over a four-hour period while controlling the temperature at 125°C. Concurrently, the initiator mixture consisting of t-amyl peroxyacetate (Lupersol 555M60) initiator dissolved in pentyl propionate was fed to the reaction flask via a second piston pump over the same time period. The two feeds were introduced into the reactor below the liquid surface and from opposite sides of the reactor. After completion of the feeding step, the monomer line was flushed with pentyl propionate and the reaction was allowed to proceed for 30 minutes at 125°C. Then a second initiator feed consisting of a mixture of t-amyl peroxyacetate dissolved in pentyl propionate was fed to the reaction mass and the reaction was allowed to proceed for an additional 2 hours at 125°C. The solution of copolymer was then cooled to room temperature and analyzed by gel permeation chromatography using polystyrene standards to determine relative average molecular weights, for total solids content, and for viscosity. The results indicated that Example 1 copolymer/oligomer prepared from Preparation A hydroxyalkyl methacrylate had a lower molecular weight and lower viscosity (Brookfield viscosity), characteristics which allow preparation of higher total solids coating formulations, than Control Examples A and B copolymers/oligomers.

|  | Example | Control Examples | |
|---|---|---|---|
|  | 1 | A | B |
| Initial pentyl propionate solvent, g | 100.0 | 100.0 | 100.0 |
| **Monomer Mixture, g(mol)** | | | |
| Preparation A hydroxy-alkyl methacrylate | 120.0(0.56) | --- | --- |
| Hydroxyethyl methacrylate --- | | 72.9(0.56) | --- |
| Hydroxypropyl methacrylate | --- | --- | 80.7(0.56) |
| Butyl acrylate | 90.0(0.62) | 113.1(0.78) | 109.5(0.75) |
| Methyl methacrylate | 90.0(0.90) | 114.0(1.14) | 108.5(1.09) |
| 3-Mercapto-1-propanol | 1.80 | 1.80 | 1.80 |
| **Initiator Mixture, g** | | | |
| Pentyl propionate | 62.7 | 62.7 | 62.7 |
| t-Amylperoxyacetate | 18.3 | 18.3 | 18.3 |
| **Monomer Line Flush** | | | |
| Pentyl propionate, g | 15.0 | 15.0 | 15.0 |
| **Second Initiator Mixture, g** | | | |
| Pentyl propionate | 15.0 | 15.0 | 15.0 |
| t-Amylperoxyacetate | 1.7 | 1.7 | 1.7 |
| **Copolymer Properties** | | | |
| Total Solids, % | 56.92 | 58.41 | 59.38 |
| $M_n$ | 2,383 | 2,778 | 3,191 |
| $M_w$ | 6,306 | 9,569 | 8,215 |
| $M_w/M_n$ | 2.64 | 3.44 | 2.57 |
| Viscosity, cP(°C) | 630(20°C) | 1,130(21°C) | 1,190(21°C) |

Example 2 and Control Example C. The copolymer/oligomer of Example 1 and that of Control Example A were formulated into thermally-curable coating systems by weighing the ingredients identified below into glass containers, stirring well, and applying to 4-inch x 6-inch Bonderite-952 steel panels using a 10-mil (254-micron) wet-clearance applicator. The coated panels were thermally cured in a 140°C circulating-air oven for 30 minutes. Several panels of each coating system were prepared in this manner. The results indicated that the coating of Example 2 had improved hardness, crosshatch adhesion, and acid etch resistance at pH 3.0 in comparison to the coating of Control Example C.

| Ingredients, grams | Example 2 | Control Example C |
|---|---|---|
| Example 1 oligomer | 10.0 | ---- |
| Control Example A oligomer | ----- | 10.0 |
| Aminoplast 1 | 2.0 | 2.0 |
| Surfactant 1 | 0.16 | 0.16 |
| Surfactant 2 | 0.16 | 0.16 |
| Methyl amyl ketone solvent | 1.0 | 1.0 |
| Catalyst 1 | 0.05 | 0.05 |
| | | |
| Cured Coating Properties | | |
| Film Thickness | 2.3 | 2.3 |
| Double rubs | >100 | >100 |
| 60° Gloss | 95.3 | 95.8 |
| 20° Gloss | 83.9 | 82.4 |
| Pencil hardness | 3H | 2H |
| Crosshatch adhesion | 5B | 4B |
| Impact resistance, in.lbs., forward/reverse | 40/2 | 40/2 |
| | | |
| Acid etch resistance | | |
| pH 2.0, 30 minutes, at 50°C | 4/4 | 3/3 |
| at 60°C | 3/3 | 1/1 |
| Temperature first visible defect, °C | 48/48 | 40/40 |
| Temperature first severe defect, °C | 68/68 | 7/60 |
| Water spot test | 5/5 | 5/5 |

Example 3 and Control Example D. The copolymer/oligomer of Example 1 and that of Control Example A were formulated into thermally-curable coating systems and applied a similar manner as in Example 2 and Control Example C except different amounts of catalyst and aminoplast were used. The coated panels were thermally cured at different (lower) temperatures than were used for Example 2 and Control Example C to determine the effect of cure temperature on properties. Cure time was 30 minutes. Several panels of each coating system were prepared in this manner. The results indicated that the coating of Example 3 had equivalent solvent resistance, equivalent or better hardness, and markedly improved acid-etch resistance than the coating of Control Example D.

14

| Ingredients, grams | Example 3 | Control Example D |
|---|---|---|
| Example 1 oligomer | 10.0 | ---- |
| Control Example A oligomer | ---- | 10.0 |
| Aminoplast 1 | 1.90 | 1.95 |
| Surfactant 1 | 0.16 | 0.16 |
| Surfactant 2 | 0.16 | 0.16 |
| Methyl amyl ketone solvent | 1.0 | 1.0 |
| Catalyst 1 | 0.31 | 0.31 |

| Cured Coating Properties | Example 3 | | Control Example D | |
|---|---|---|---|---|
| Cure temperature, °C | 110 | 125 | 110 | 125 |
| Double rubs | >100 | >100 | >100 | >100 |
| Pencil hardness | F | H | F | F |

**Acid etch resistance**

| | Example 3 | | Control Example D | |
|---|---|---|---|---|
| pH = 2.0, 30 minutes | | | | |
| contact at 50°C | 3/3 | 3/3 | 3/3 | 2/2 |
| at 60°C | 1/2 | 2/2 | 1/2 | 1/1 |
| Temperature first visible defect, °C | 47/47 | 50/50 | 45/48 | 49/49 |
| Temperature first severe defect, °C | 60/62 | 5/65 | 60/62 | 3/53 |
| 10% acid, 15 minutes contact | | | | |
| at 50°C | 5/5 | 5/5 | 5/5 | 5/5 |
| at 60°C | 4/4 | 5/5 | 1/1 | 1/1 |
| Temperature first noticeable defect, °C | 58/58 | 65/67 | 56/58 | 53/53 |
| Temperature first severe defect, °C | 62/64 | 70/70 | 60/60 | 60/60 |

Example 4 and Control Example E. The copolymer/oligomer of Example 1 and that of Control Example A were formulated into thermally-curable, isocyanate crosslinked coating systems by weighing the ingredients identified below into glass containers, stirring well, and applying to 4-inch x 6-inch Bonderite-952 steel panels using a 10-mil (254-micron) wet-clearance applicator. The coated panels were thermally cured in a 140°C circulating-air oven for 45 minutes. Several panels of each coating system were prepared in this manner. The results indicated that the coating of Example 4 had improved adhesion, 60° gloss, 20° gloss and improved or equivalent acid etch resistance at pH 2.0 than the coating of Control Example E.

15

| Ingredients, grams | Example 4 | Control Example E |
|---|---|---|
| Example 1 oligomer | 10.0 | ---- |
| Control Example A oligomer | ---- | 10.0 |
| Blocked Isocyanate 1 | 5.0 | 5.0 |
| Surfactant 1 | 0.20 | 0.20 |
| Catalyst 2 | 0.20 | 0.20 |
| Methyl amyl ketone, solvent | 2.0 | 2.0 |
| | | |
| **Cured Coating Properties** | | |
| Film Thickness | 1.7 | 1.6 |
| Double rubs | >100 | >100 |
| 60° Gloss | 97.6 | 82.7 |
| 20° Gloss | 75.8 | 55.6 |
| Pencil hardness | F | F-H |
| Crosshatch adhesion | 5B | 4B |
| Impact resistance, in.lbs., forward/reverse | 160/70 | 160/120 |
| | | |
| **Acid etch resistance** | | |
| pH = 2.0, 30 minutes contact | | |
| at 50°C | 4/4 | 4/4 |
| at 60°C | 3/3 | 2/3 |
| Temperature first visible defect, °C | 53/54 | 50/50 |
| Temperature first severe defect, °C | 64/65 | 64/64 |

Example 5. In a similar manner as described in Example 1, a copolymer/oligomer of higher viscosity was prepared by reacting 110.8 grams (0.52 moles) of Preparation A hydroxyalkyl methacrylate, 35.6 grams (0.25 moles) of butyl acrylate, and 53.6 grams (0.54 moles) of methyl methacrylate in 100 grams of pentyl propionate (solvent) and in the presence of 0.14 grams of 3-mercapto-1-propanolchain-transfer agent. The initiator feed was 6.7 grams of t-amyl peroxyacetate catalyst in 63.3 grams of solvent, the post initiator feed was 0.5 grams of chain transfer catalyst in 15 grams of solvent, and the monomer-line flush was 15 grams of solvent.

The resulting oligomer/polymer had a number average molecular weight of 2,991, a weight average molecular weight of 8,334, a polydispersity of 2.79, and a viscosity of 2,090 centipoise.

Example 6. In a similar manner as described in Example 5, a copolymer/oligomer was prepared by reacting 60.0 grams of styrene, 120.0 grams of Preparation A hydroxyalkyl methacrylate, 120.0 grams of 2-ethylhexyl methacrylate, 6.0 grams of methacrylic acid, and 1.80 grams of 3-mercapto-1-propanol in 95 grams of pentyl propionate (solvent). The initiator feed was 10 grams of t-amyl peroxyacetate catalyst in 70 grams of solvent, the post initiator feed was 0.9 grams of chain transfer catalyst in 15 grams of solvent, and the monomer-line flush was 15 grams of solvent.

The resulting oligomer/polymer had a number average molecular weight of 5,557, a weight average molecular weight of 11,342, a polydispersity of 2.04, and a viscosity of 738 centipoise.

Example 7 and Control Example F. Example 7 copolymer/oligomer was prepared from a 300-grams of a mixture of the Preparation A alkyl monomethacrylate mixture, vinyl pivalate, and vinyl 2-ethylhexanoate in the amounts described below. For comparison purposes, Control Example F was prepared from a mixture containing hydroxyethyl methacrylate, vinyl pivalate, and vinyl 2-ethylhexanoate. The copolymers were prepared in a similar manner as described in Example 1 except the quantities of materials indicated below were used, and the initiator was t-butyl peroxybenzoate. The results indicated that Example 7 copolymer prepared from Preparation A hydroxyalkyl methacrylate had a lower molecular weight and viscosity than that of Control Example F, properties which allow preparation of higher total solids coating formulations. The total solids of Control Example F oligomer were about 2% higher than those of Example 7 oligomer, which would have an effect on the measured viscosity difference.

|  | Example 7 | Control Example F |
|---|---|---|
| Initial pentyl propionate solvent, g | 100.0 | 100.0 |
| **Monomer Mixture, g** | | |
| Preparation A hydroxy-alkyl methacrylate | 60.0 | ---- |
| Hydroxyethyl methacrylate---- | | 60.0 |
| Vinyl privalate | 180.0 | 180.0 |
| Vinyl 2-ethylhexanaoate | 60.0 | 60.0 |
| 3-Mercapto-1-propanol | 0.29 | 1.74 |
| **Initiator Mixture, g** | | |
| Pentyl propionate | 70.0 | 70.0 |
| t-Butylperoxybenzoate | 11.0 | 11.0 |
| **Monomer Line Flush** | | |
| Pentyl propionate, g | 15.0 | 15.0 |
| **Second Initiator Mixture, g** | | |
| Pentyl propionate | 15.0 | 15.0 |
| t-Butylperoxybenzoate | 1.0 | 1.0 |
| **Copolymer Properties** | | |
| Total Solids, % | 60.84 | 62.66 |
| $M_n$ | 1,802 | 2,114 |
| $M_w$ | 4,892 | 5,027 |
| $M_w/M_n$ | 2.71 | 2.38 |
| Viscosity, cP | 183 | 260 |

Examples 8, 9 and 10 and Control Examples G and H. The copolymer/oligomer of Example 7 and that of Control Example F were formulated into thermally-curable, aminoplast crosslinked coating systems by weighing the ingredients described below into glass containers, stirring well, and applying to 4-inch x 6-inch Bonderite-952 steel panels using a 10-mil (254-micron) wet-clearance applicator. Before using for formulation, the Control Example F oligomer was concentrated to a total solids of 79.3% by weight by removal of pentyl propionate solvent. The coated panels were thermally cured in a 140°C circulating-air oven for 30 minutes. Several panels of each coating system were prepared in this manner.

## Examples and Control Examples

| | 8 | G | 9 | 10 | H |
|---|---|---|---|---|---|
| **Ingredients, grams** | | | | | |
| Example 9 oligomer | 10.0 | --- | 10.0 | 10.0 | ---- |
| Control Example H oligomer | ---- | 10.0 | ---- | ---- | 10.0 |
| Aminoplast 1 | 0.60 | 2.07 | ---- | ---- | ---- |
| Aminoplast 2 | ---- | ---- | 1.41 | 2.0 | 2.0 |
| Surfactant 1 | 0.13 | 0.20 | 0.16 | 0.16 | 0.16 |
| Surfactant 2 | 0.14 | 0.20 | 0.16 | 0.16 | 0.16 |
| Catalyst 1 | 0.14 | 0.32 | 0.05 | 0.05 | 0.05 |
| Methyl amyl ketone, solvent | ---- | 2.5 | ---- | ---- ---- | |
| Oligomer hydroxyl to aminoplast alkyloxy molar ratio | 1/2.74 | 1/2.74 | 1/2.74 | 1/3.97 | 1/2.4 |
| **Cured Coating Properties** | | | | | |
| Film Thickness | 2.5 | 2.3 | 2.2 | 2.5 | 2.1 |
| Double rubs | 100 | >100 | >100 | >100 | >100 |
| 60° Gloss | 86.7 | 96.4 | 96.2 | 97.1 | 93.9 |
| 20° Gloss | 74.1 | 84.6 | 79.4 | 84.9 | 79.7 |
| Pencil hardness | 2B | H | HB | F | F |
| Crosshatch adhesion | 1B | 4B | 5B | 5B | 4B-5B |
| Impact resistance in.lbs., forward/reverse | 4/2 | 40/2 | 30/2 | 40/2 | 40/2 |
| **Acid etch resistance** | | | | | |
| pH = 2.0, 30 minutes contact | | | | | |
|    at 50°C | 4/4 | 3/3 | 5/5 | 3/3 | 1/1[+] |
|    at 60°C | 2/1 | /1 | 3/3 | 1/1 | 1/1 |
| Temperature first visible defect, °C | 45/47 | 45/48 | 52/52 | 50/50 | 50/50 |
| Temperature first severe defect, °C | 62/60 | 60/60 | 66/66 | 58/60 | 50/50 |
| 10% acid, 15 minutes contact | | | | | |
|    at 50°C | 5/5 | 5/5 | 5/5 | 5/5 | 5/5 |
|    at 60°C | 4/4 | 1/1[+] | 4/4 | 5/5 | 4/4 |
| Temperature first visible defect, °C | 60/60 | 55/55 | 60/62 | 62/64 | 62/62 |
| Temperature first severe defect, °C | 64/64 | 58/58 | 70/70 | 70/70 | 65/65 |

[+]Coating was cut through to bare steel, i.e., very severe attack.

Example 11. A 4-necked glass reactor is equipped with a stirrer, temperature-measuring device, gas sparge, condenser, and a feeding port. Two moles (228 grams) of e-caprolactone are added to the reactor and heated to and held at 100°C for 30 minutes while sparging with dry nitrogen. The temperature is then increased to 130°C and the sparge is changed to dry air. Then, one mole (214 grams) of the Preparation A methacrylate ester is added along with 500 parts per million of the monomethylether of hydroquinone and 50 parts per million of stannous octanoate. The reaction mass is held at 130-135°C for 6 hours after which time it is cooled to room temperature. This caprolactone acrylate is stored for future use.

Example 12. One hundred grams (0.226 moles or equivalents) of the caprolactone acrylate of Example 11 is placed in the Example 11 reactor and heated to 45°C while maintaining an air sparge. Then 29.6 grams (0.113 moles or 0.226 equivalents) of 4,4'-dicyclohexylmethyl diisocyanate are added. A small exotherm is noted. The reaction is allowed to proceed at 45°C-50°C for 10 hours after which time the urethane acrylate reaction product is cooled to room temperature and stored with an air blanket for later use in radiation-cure formulations.

Example 13. One hundred grams (0.467 moles or equivalents) of the Preparation A methacrylate ester are placed in the Example 11 reactor. The acrylate ester is heated to 45°C while maintaining an air sparge. Then 61.2 grams (0.234 moles or 0.467 equivalents) of 4,4'-dicyclohexylmethyl diisocyanate are added. A small exotherm is noted. The reaction is allowed to proceed at 45°C-50°C for 10 hours after which time the urethane acrylate reaction product is cooled to room temperature and stored with an air blanket for later use in radiation-cure formulations.

Example 14. To an amber-colored glass container, 20 grams of the acrylic oligomer of Example 1,40 grams of 3,4-epoxycyclohexyl 3,4-epoxycyclohexane carboxylate, and 1.8 grams of Photoinitiator 1 are added. The ingredients are well mixed and then applied to a steel panel by the draw-down method. The coated panel is then placed on a conveyor moving at 30 feet/minute and passing under a 300 watt-per-inch medium-pressure mercury vapor lamp to effect cure. A tack-free, clear coating results.

Example 15. Ten grams of the acrylate oligomer of Example 5, 30 grams of 3,4-epoxycyclohexyl 3,4-epoxycyclohexane carboxylate, 1.5 grams of diethylammonium triflate catalyst, and 5 grams of methyl amyl ketone solvent are added to a glass container and well mixed. The mixture is coated onto a steel panel with a No. 22 wire-wound rod. The coated panel is allowed to air dry for 10 minutes and then it is oven baked at 115°C for 20 minutes. A clear, tack-free coating with good water resistance results.

Example 16. In a similar manner as described in Example 5, a copolymer/oligomer was prepared by placing 100 grams of pentyl propionate in the reactor and adding 100.50 grams of isodecyl methacrylate, 115 grams of the Preparation A hydroxyalkyl acrylate, about 5 grams of a diacrylate with the following structure,

$$CH_2 = C(CH_3)\text{-}CO\text{-}O\text{-}CH_2 C(CH_3)_2 CH(O\text{-}OC\text{-}C(CH_3) = CH_2)CH(CH_3)CH_3,$$

73.50 grams of isobornyl methacrylate, 6 grams of methacrylic acid, and 0.21 grams of 3-mercapto-1-propanol. The initiator feed was composed of 10 grams of t-amyl peroxyacetate dissolved in 70 grams of pentyl propionate, the post initiator feed was composed of 0.9 grams of t-amyl peroxyacetate dissolved in 15 grams of pentyl propionate, and the monomer line flush was 15 grams of pentyl propionate. The resulting polymer had a Brookfield viscosity of 715 cP at a total solids content of 54.58%, which indicated it would be useful for preparing high solids coatings.

Examples 17 and 18. These copolymers were prepared from a 300-gram mixture of a Preparation A alkyl monomethacrylate mixture, and other copolymerizable ethylenically unsaturated monomers as indicated below. The initial butyl propionate solvent was placed in a 2-liter, four-neck, glass reaction flask equipped with a mechanical stirrer, a Thermo-watch heat controller, a nitrogen sparger, a water-cooled condenser, and 500-milliliter and 125-milliliter addition funnels. A nitrogen sparge was maintained throughout the procedure. The solvent was heated to 140°C, and the monomer mixture was fed by means of a piston pump to the flask over a four-hour period while controlling the temperature at 140°C. Concurrently, the initiator mixture consisting of t-amyl peroxyacetate initiator dissolved in butyl propionate was fed to the reaction flask by a second piston pump over the same time period. The two feeds were introduced into the reactor below the liquid surface and from opposite sides of the reactor. After completion of the feeding step, the monomer line was flushed with 15g butyl propionate and the reaction was allowed to proceed for 30 minutes at 140°C. Then a second initiator feed consisting of a mixture of 2,5-dimethyl-2,5-di(2-ethylhexanoyl-peroxy)hexane dissolved in butyl propionate was fed to the reaction mass and the reaction was allowed to proceed for an additional 2 hours at 140°C. The solution of copolymer was then cooled to room temperature and analyzed by gel permeation chromatography using polystyrene standards to determine relative average molecular weights, for total solids content, and for viscosity. In each case, low viscosity products that were useful in coating formulations were prepared.

|  | Examples | |
|---|---|---|
|  | 17 | 18 |
| Initial butyl propionate solvent, g | 100.0 | 100.0 |
| **Monomer Mixture, g(mol)** | | |
| Preparation A hydroxy-alkylmethacrylate | 120.0 | 120.0 |
| Lauryl methacrylate | 105.0 | 105.0 |
| t-Butyl methacrylate | 69.0 | --- |
| Methyl methacrylate | --- | 69.0 |
| Methacrylic acid | 6.0 | 6.0 |
| **Initiator Mixture, g** | | |
| Butyl propionate | 70.0 | 70.0 |
| t-Amylperoxyacetate* | 10.0 | 10.0 |
| **Monomer Line Flush** | | |
| Pentyl propionate, g | 15.0 | 15.0 |
| **Second Initiator Mixture, g** | | |
| Pentyl propionate | 15.0 | 15.0 |
| 2,5-Dimethyl-2,5-di(2-ethyl hexanoylperoxy)hexane** | 0.9 | 0.9 |
| **Copolymer Properties** | | |
| Total Solids, % | 55.1 | 56.7 |
| $M_n$ | 4528 | 5397 |
| $M_w$ | 9285 | 10,620 |
| $M_w/M_n$ | 2.05 | 1.97 |
| **Viscosity+, cP, 25°C** | 299 | 717 |

\* Lupersol 555M60(60TS)
\*\*Lupersol 256
+ Brookfield viscosity

Example 19. The copolymer of Example 18 is reacted with butyl isocyanate using 75% of the moles of butyl isocyanate required for reaction with the available hydroxyl groups on the polymer. The butyl isocyanate is slowly added to the copolymer in a suitable enclosed reactor equipped with a stirrer and other conventional equipment at room temperature over a 30-minute time period. The reaction mass undergoes a mild exotherm shortly after the addition. After 8 hours, infrared analysis is used to analyze the reaction mass. The reaction is continued until the butyl isocyanate is reduced to a desired level. The modified polymer containing N-butyl carbamoyloxyalkanoyoxyalkyl groups is stripped of any residual isocyanate and then stored for future use as an intermediate in preparing coating compositions.

Example 20. Fifty grams (0.233 moles or equivalents) of the Preparation A methacrylate ester are placed in a reactor equipped with a stirrer, feeding port, and means of temperature measurement and control. The methacrylate ester is heated to 80°C and 0.23 equivalents of phthalic anhydride are slowly added. The temperature is then increased to 120°C and held there for 2 hours after which time the reaction mass is cooled to room temperature and stored for future use.

Although the invention has been illustrated by certain of the preceding examples, it is not to be construed as being limited thereby; but rather, the invention encompasses the generic area as hereinbefore disclosed. Various modifications and embodiments can be made without departing from the spirit and scope thereof.

**Claims**

1. A compound represented by the formula:

$$R_1 R_2 C = C(R_3) - C(O) - O - R_4$$

wherein:

$R_1$, $R_2$ and $R_3$ are the same or different and are hydrogen or a substituted or unsubstituted monovalent hydrocarbon residue;

$R_4$ is a substituted or unsubstituted monovalent hydrocarbon residue represented by the formula:

$$-(CR_5 R_6)_v - (CR_7 R_8)_w - (C(OH)R_9)_x - (CR_{10}R_{11})_y - (CR_{12}R_{13})_z - R_{14}$$

wherein:

each $R_5$, $R_6$, $R_7$, $R_8$, $R_9$, $R_{10}$, $R_{11}$, $R_{12}$ and $R_{13}$ are the same or different and are hydrogen or a substituted or unsubstituted monovalent hydrocarbon residue;

$R_{14}$ is hydrogen, hydroxyl or a substituted or unsubstituted monovalent hydrocarbon residue provided $R_{14}$ is hydroxyl when x is value of 0 and $R_{14}$ is other than hydroxyl when x is a value of 1;

each of v, w, y and z is a value of from 0 to about 5 and the sum of v + w + x + y + z is a value of from about 3 to about 15; and

x is a value of 0 or 1;

provided (i) at least one of w and y is a value other than 0 when x is a value of 1; (ii) at least one of $R_7$, $R_8$, $R_9$, $R_{10}$ and $R_{11}$ is other than hydrogen when x is a value of 1; and (iii) at least one of $R_{12}$ and $R_{13}$ is other than hydrogen when x is a value of 0.

2. The compound of claim 1 which is selected from 2-ethyl-3-hydroxyhexyl methacrylate, 1-propyl-2-ethyl-3-hydroxypropylmethacrylate, 1-ethyl-2-methyl-3-hydroxypropyl methacrylate, 2-methyl-3-hydroxypentyl methacrylate, 2-propyl-3-hydroxyheptyl methacrylate, 1-butyl-2-propyl-3-hydroxypropyl methacrylate, 2-ethyl-3-hydroxyheptyl methacrylate, 1-butyl-2-ethyl-3-hydroxypropyl methacrylate, 2-propyl-3-hydroxypropyl methacrylate, 2-ethyl-3-hydroxypropyl methacrylate, 1-i-butyl-2-i-propyl-3-hydroxypropyl methacrylate, 2-i-propyl-3-hydroxy-5-methylhexyl methacrylate, 1-methyl-2-i-propyl-3-hydroxypropyl methacrylate, 2-i-propyl-3-methyl-3-hydroxypropyl acrylate, 1-i-butyl-3-hydroxypropyl methacrylate, 3-hydroxy-5-methylhexyl methacrylate, 1-methyl-2-butyl-3-hydroxypropyl methacrylate, 2-butyl-3-hydroxybutyl methacrylate, 1-i-propyl-2,2-dimethyl-3-hydroxypropyl methacrylate, and 2,2-dimethyl-3-hydroxy-4-methylpentyl methacrylate.

3. A process for producing the compound of claim 1 which is selected from:
   (i) an esterification process comprising reacting a diol compound with methacrylic acid or acrylic acid to produce the compound;
   (ii) a process comprising reacting a diol compound with methacrylic anhydride or acrylic anhydride to produce the compound; or
   (iii) a transesterification process comprising reacting a diol compound with alkyl methacrylate or alkyl acrylate to produce the compound.

4. The process of claim 3 further comprising derivatizing the compound in which the derivatizing reaction comprises an oxidation, alkoxylation, carboxylation, reduction, carbamoylation, hydrogenation, dehydrogenation, condensation, amination, esterification, etherification, silylation, alkylation or acylation reaction.

5. The product produced by the process of claims 3 or 4.

6. A reactive surfactant comprising an alkoxylated derivative of a compound of claim 1 or a wet adhesion monomer comprising a product produced by the process of claim 3.

7. A copolymer comprising the reaction product of (a) one or more hindered-hydroxyl functional (meth)-acrylate monomers and (b) at least one other monomer copolymerizable therewith, said copolymer having (i) a number average molecular weight of less than about 12,000, (ii) a glass transition temperature of from -30°C to about 100°C, and (iii) a styrene concentration of from 0 to less than about 20 weight percent, and said hindered-hydroxyl functional (meth)acrylate monomer having a diacrylate content of less than about 5 weight percent.

8. The copolymer of claim 7 in which the hindered-hydroxyl functional (meth)acrylate monomer is a compound of claim 1.

9. The copolymer of claim 7 in which the hindered-hydroxyl functional (meth)acrylate monomer is selected from one or more of 2-ethyl-3-hydroxyhexyl methacrylate, 1-propyl-2-ethyl-3-hydroxypropyl methacrylate, 1-ethyl-2-methyl-3-hydroxypropyl methacrylate, 2-methyl-3-hydroxypentyl methacrylate, 2,2,4-trimethyl-3-hydroxypentyl methacrylate, 2-propyl-3-hydroxyheptyl methacrylate, 1-butyl-2-propyl-3-hydroxypropyl methacrylate, 2-ethyl-3-hydroxyheptyl methacrylate, 1-butyl-2-ethyl-3-hydroxypropyl methacrylate, 2-propyl-3-hydroxypropyl methacrylate, 2-ethyl-3-hydroxypropyl methacrylate, 1-i-butyl-2-i-propyl-3-hydroxypropyl methacrylate, 2-i-propyl-3-hydroxy-5-methylhexyl methacrylate, 1-methyl-2-i-propyl-3-hydroxypropyl methacrylate, 2-i-propyl-3-methyl-3-hydroxypropyl acrylate, 1-i-butyl-3-hydroxypropyl methacrylate, 3-hydroxy-5-methylhexyl methacrylate, 1-methyl-2-butyl-3-hydroxypropyl methacrylate, 2-butyl-3-hydroxybutyl methacrylate, 1-i-propyl-2,2-dimethyl-3-hydroxypropyl methacrylate, and 2,2-dimethyl-3-hydroxy-4-methylpentyl methacrylate.

10. The copolymer of claim 7 in which the other copolymerizable monomer is selected from one or more other (meth)acrylates, hydroxyalkyl (meth)acrylates and vinyl compounds.

11. A composition comprising (a) a hindered-hydroxyl functional (meth)acrylate-containing copolymer of claim 7 and (b) a crosslinking agent.

12. The composition of claim 11 in which the hindered-hydroxyl functional (meth)acrylate monomer is selected from one or more of 2-ethyl-3-hydroxyhexyl methacrylate, 1-propyl-2-ethyl-3-hydroxypropyl methacrylate, 1-ethyl-2-methyl-3-hydroxypropyl methacrylate, 2-methyl-3-hydroxypentyl methacrylate, 2,2,4-trimethyl-3-hydroxypentyl methacrylate, 2-propyl-3-hydroxyheptyl methacrylate, 1-butyl-2-propyl-3-hydroxypropyl methacrylate, 2-ethyl-3-hydroxyheptyl methacrylate, 1-butyl-2-ethyl-3-hydroxypropyl methacrylate, 2-propyl-3-hydroxypropyl methacrylate, 2-ethyl-3-hydroxypropyl methacrylate, 1-i-butyl-2-i-propyl-3-hydroxypropyl methacrylate, 2-i-propyl-3-hydroxy-5-methylhexyl methacrylate, 1-methyl-2-i-propyl-3-hydroxypropyl methacrylate, 2-i-propyl-3-methyl-3-hydroxypropyl acrylate, 1-i-butyl-3-hydroxypropyl methacrylate, 3-hydroxy-5-methylhexyl methacrylate, 1-methyl-2-butyl-3-hydroxypropyl methacrylate, 2-butyl-3-hydroxybutyl methacrylate, 1-i-propyl-2,2-dimethyl-3-hydroxypropyl methacrylate, and 2,2-dimethyl-3-hydroxy-4-methylpentyl methacrylate.

13. The composition of claim 11 in which the other copolymerizable monomer is selected from one or more other (meth)acrylates, hydroxyalkyl (meth)acrylates and vinyl compounds.

14. The composition of claim 11 in which the crosslinking agent is selected from aminoplasts, multifunctional isocyanates, phenolics, cycloaliphatic epoxides, glycidyl epoxides, carbodiimides and polycarbodiimides.

15. A method of protecting a surface from corrosion when exposed to an acidic or alkaline environment which comprises applying to the surface a composition comprising (a) a hindered-hydroxyl functional (meth)acrylate-containing copolymer and (b) a crosslinking agent, and curing said composition.

16. A curable coating composition comprising the copolymer of claim 7 and a crosslinking agent, and a cured film prepared therefrom.

17. The curable coating composition of claim 16 which is cured with actinic or thermal energy or a mixture thereof.

18. An adhesive ink or sealant composition comprising the copolymer of claim 7 and a crosslinking agent, or a water-borne, solvent-borne, powdered, colored or clear coating comprising the copolymer of claim 7.

**European Patent Office**

**EUROPEAN SEARCH REPORT**

Application Number

EP 93 11 6804

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | GB-A-1 092 884 (EASTMAN KODAK COMPANY) * the whole document * | 1-3,15 | C07C69/54 C08F220/28 C08F8/00 |
| X | PATENT ABSTRACTS OF JAPAN vol. 13, no. 224 (C-599)(3572) & JP-A-01 034 947 (KYOWA GAS CHEM IND KK) * abstract * | 1-3 | C09D133/06 C08G18/80 |
| X | EP-A-0 225 808 (EXXON CORP) * page 8, line 34 - line 47; table 5 * | 1-3,7, 9-16 | |
| X | US-A-3 256 225 (NEVIN) * formula column 1 * | 1 | |

**TECHNICAL FIELDS SEARCHED** (Int.Cl.5)

C08F
C09D

The present search report has been drawn up for all claims

| Place of search | Date of completion of the search | Examiner |
|---|---|---|
| THE HAGUE | 27 January 1994 | Schueler, D |